# EUROPEAN PATENT APPLICATION

(11) **EP 3 828 262 A1**
(43) Date of publication of application: **02.06.2021**
(21) Application number: 19840012.9
(22) Date of filing: 22.07.2019
(51) Int. Cl.: C12N 5/00, C07K 16/28, C12N 5/071, C12Q 1/6881, G01N 33/48, G01N 33/53

(54) **NOVEL RENAL PROGENITOR CELL MARKER AND METHOD FOR CONCENTRATING RENAL PROGENITOR CELLS USING SAME**

(30) Priority: 23.07.2018 JP 2018138040
(71) Applicant: Kyoto University, Kyoto-shi, Kyoto 606-8501 (JP)
(72) Inventor: OSAFUNE, Kenji, Kyoto-shi, Kyoto 606-8501 (JP); ARAOKA, Toshikazu, Kyoto-shi, Kyoto 606-8501 (JP); WATANABE, Akira, Kyoto-shi, Kyoto 606-8501 (JP)
(74) Representative: Hoffmann Eitle
(86) International application number: PCT/JP2019/028648
(87) International publication number: WO 2020/022261

(57) **Abstract**

To concentrate renal progenitor cells by extracting MET-positive cells and/or AGTR2-positive cells from a renal progenitor cell-containing cell population obtained by, for example, induction from pluripotent stem cells.

## Description

### Technical Field

The present invention relates to methods for concentrating and detecting renal progenitor cells and kits for use in those methods. The invention also relates to a method for producing renal progenitor cells from pluripotent stem cells.

### Background Art

Currently, the number of patients with chronic kidney disease (CKD) is estimated to be about 13,000,000 in Japan, and it is called a new national disease. There are few curative treatments for chronic kidney disease, and the number of patients with end-stage chronic renal failure who require dialysis therapy due to the disease progression is more than 320,000, which is a major problem not only medically but also medical-economically. Kidney transplantation is one of the radical treatments for chronic kidney disease including end-stage chronic renal failure, but supply is not keeping up with demand due to a serious shortage of donor organs.

In order to solve the shortage of donor organs in kidney transplantation and to develop a new cell therapy for CKD, it is necessary to establish a method for producing renal cells or renal tissue from induced pluripotent stem (iPS) cells with high efficiency.

The kidney is derived from the intermediate mesoderm, which is an early embryonic tissue. In vertebrates, the intermediate mesoderm forms three kidneys, which are pronephros, mesonephros, and metanephros, and in mammals, the metanephros becomes the adult kidney. The metanephros occurs through the interaction of two tissues which are a tissue called mesenchyme that will differentiate into nephrons and stroma of the adult kidney, and a tissue called ureteral bud that will differentiate into the lower renal pelvis, ureter, and part of the bladder from the collecting duct of the adult kidney (Non Patent Literature 1 and 2). Further, in recent years, it has been reported that the intermediate mesoderm is divided into two parts, anterior and posterior parts, and the ureteral bud develops from the anterior intermediate mesoderm and the mesenchyme develops from the posterior intermediate mesoderm (Non Patent Literature 3).

Methods for inducing renal progenitor cell differentiation from human iPS cells and human embryonic stem (ES) cells have been developed (Non Patent Literature 3 to 6). However, since the differentiation induction efficiency is not 100%, unintended cells such as undifferentiated iPS cells and cells of other organ lineages are included in the differentiation culture. Use of renal progenitor cells containing unintended cells for transplantation therapy may cause adverse events such as tumorigenesis, and also use of such cells for disease model creation or drug nephrotoxicity evaluation system construction, it is inefficient because they include unintended cells, and there is a risk that the experimental results will be influenced. It is therefore necessary to establish a method for selectively isolating only renal progenitor cells obtained by differentiation induction from human iPS cells.

The present inventors have recently reported a method for isolating renal progenitor cells induced to differentiate from human iPS cells using a unique combination of cell surface antigens (Non Patent Literature 7 and Patent Literature 1). Meanwhile, there was room for improvement in terms of isolation/concentration efficiency.

### Citation List

### Patent Literature

Patent Literature 1: WO2017/043666

### Non Patent Literature

Non Patent Literature 1: Osafune K., et al., Development 2006; 133: 151-61.
Non Patent Literature 2: Kobayashi A., et al., Cell Stem Cell 2008; 3: 169-81.
Non Patent Literature 3: Taguchi A., et al., Cell Stem Cell. 2014; 14: 53-67.
Non Patent Literature 4: Takasato M., et al., Nat Cell Biol. 2014; 16: 118-26.
Non Patent Literature 5: Takasato M., et al., Nature. 2015; 526: 564-568.
Non Patent Literature 6: Morizane R., et al., Nat. Biotechnol. 2015; 33: 1193-1200.
Non Patent Literature 7: Hoshina A., et al., Sci Rep, Sci Rep. 2018; 8: 6375.

### Summary of Invention

### Technical Problem

An object of the invention is to provide a method for efficiently isolating and purifying renal progenitor cells from a cell population containing renal progenitor cells. Solution to Problem

As a result of intensive studies to achieve the above object, the inventors found for the first time that by using MET (also known as hepatocyte growth factor receptor (HGFR)) and/or angiotensin II receptor type 2 (AGTR2) as markers, a highly pure renal progenitor cell population can be obtained and produced from a cell population containing renal progenitor cells. The invention has been completed based on such findings.

Specifically, the present invention has the following features.
[1] A method for concentrating renal progenitor cells, comprising a step of extracting MET-positive cells and/or AGTR2-positive cells from a cell population containing renal progenitor cells.
[2] The method according to [1], wherein the renal progenitor cells are OSR1 (odd-skipped related 1)- and SIX2 (also known as SIX Homeobox 2)-positive.
[3] The method according to [1] or [2], wherein the step of extracting MET-positive cells and/or AGTR2-positive cells is performed using an anti-MET antibody and/or an anti-AGTR2 antibody.
[4] The method according to any one of [1] to [3], wherein the renal progenitor cells are human renal progenitor cells.
[5] The method according to any one of [1] to [4], wherein the cell population containing renal progenitor cells is a cell population containing renal progenitor cells obtained by differentiation induction from pluripotent stem cells.
[6] The method according to [5], wherein the pluripotent stem cells are ES cells or iPS cells.
[7] A method for detecting renal progenitor cells, comprising a step of detecting MET-positive cells and/or AGTR2-positive cells in a cell population containing renal progenitor cells.
[8] The method according to [7], wherein the renal progenitor cells are OSR1- and SIX2-positive.
[9] A kit for extracting or detecting renal progenitor cells, comprising a reagent that specifically binds to MET and/or a reagent that specifically binds to AGTR2.
[10] The kit according to [9], wherein the renal progenitor cells are OSR1- and SIX2-positive.
[11] The kit according to [9] or [10], wherein the reagent that specifically binds to MET is an anti-MET antibody, and the reagent that specifically binds to AGTR2 is an anti-AGTR2 antibody.
[12] A method for producing renal progenitor cells, comprising: a step of obtaining a cell population containing renal progenitor cells from pluripotent stem cells; and a step of extracting renal progenitor cells from the obtained cell population using MET and/or AGTR2 positivity as an index.
[13] The method according to [12], wherein the renal progenitor cells are OSR1- and SIX2-positive.
[14] The method according to [12] or [13], wherein the step of obtaining a cell population containing renal progenitor cells from pluripotent stem cells comprises the following steps (i) to (vi):
   (i) culturing pluripotent stem cells in a medium containing fibroblast growth factor (FGF) 2, bone morphogenetic protein (BMP) 4, a glycogen synthase kinase (GSK)-3β inhibitor, and retinoic acid or a derivative thereof;
   (ii) culturing the cells obtained in the step (i) in a medium containing FGF2, a GSK-3β inhibitor, and BMP7;
   (iii) culturing the cells obtained in the step (ii) in a medium containing FGF2, a GSK-3β inhibitor, BMP7, and a transforming growth factor (TGF) β inhibitor;
   (iv) culturing the cells obtained in the step (iii) in a medium containing FGF2, a GSK-3β inhibitor, BMP7, activin, and a Rho-kinase (ROCK) inhibitor;
   (v) culturing the cells obtained in the step (iv) in a medium containing retinoic acid or a derivative thereof and FGF9.; and
   (vi) culturing the cells obtained in the step (v) in a medium containing a GSK-3β inhibitor and FGF9.
[15] A method for producing a renal organoid, comprising: a step of inducing renal progenitor cells by the method according to any one of [12] to [14]; and a step of culturing the obtained renal progenitor cells to form a renal organoid.
[16] A renal progenitor cell marker consisting of MET and/or AGTR2.
[17] The renal progenitor cell marker according to [16], wherein renal progenitor cells are OSR1- and SIX2-positive.
[18] Use of MET and/or AGTR2 as a renal progenitor cell marker.
[19] The use according to [18], wherein the renal progenitor cells are OSR1- and SIX2-positive.
[20] An agent for treating or preventing renal diseases, comprising renal progenitor cells obtained by the method according to any one of [12] to [14] or a renal organoid obtained by the method according to [15].
[21] A method for treating or preventing renal diseases, comprising a step of administering a therapeutically or prophylactically effective amount of renal progenitor cells obtained by the method according to any one of [12] to [14] or a renal organoid obtained by the method according to [15].
[22] Use of renal progenitor cells obtained by the method according to any one of [12] to [14] or a renal organoid obtained by the method according to [15] in producing medicines for treating or preventing renal diseases.
[23] Renal progenitor cells obtained by the method according to any one of [12] to [14] or a renal organoid obtained by the method according to [15] for use in treating or preventing renal diseases.

### Advantageous Effects of Invention

According to the invention, it became possible to isolate and concentrate high-purity renal progenitor cells from a cell population in which pluripotent stem cells (e.g., iPS cells) are induced to differentiate into renal progenitor cells, by using MET and/or AGTR2 as markers. The renal progenitor cell population obtained by the method of the invention can be used for regenerative medicine for renal diseases such as renal failure. It is also expected to apply the renal progenitor cell population to organ reconstruction, drug discovery screening, and drug toxicity evaluation system development.

The metanephros is one of the fetal kidney tissues that form the adult kidney and is an essential component in kidney regeneration. In addition, since there are many renal diseases that occur in metanephric nephron progenitor cells, glomeruli derived from them, and renal tubules, it is also useful for creating a renal disease model. Accordingly, the method of the invention can also contribute from the viewpoint of searching for a therapeutic method for renal diseases.

### Brief Description of Drawings

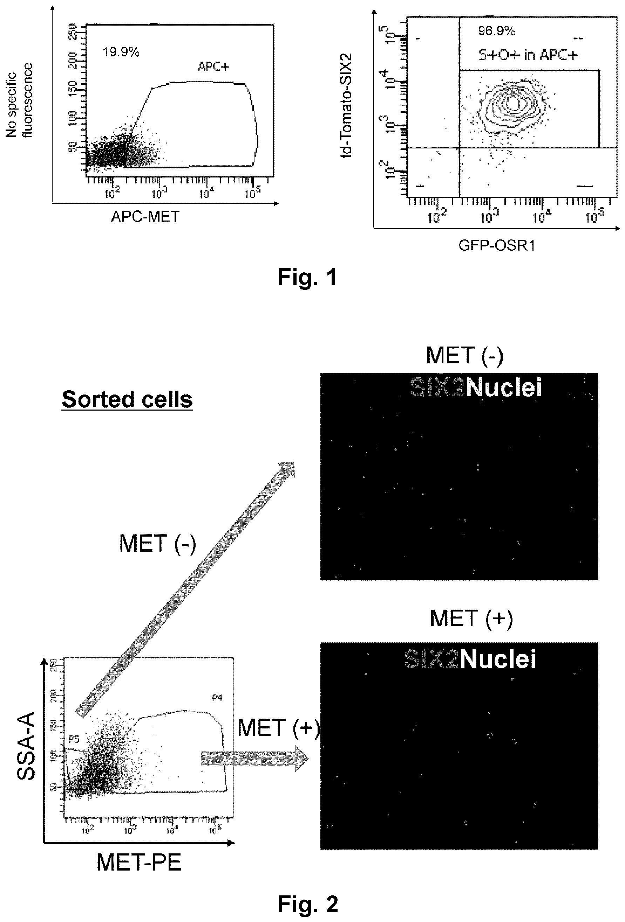

Fig. 1 shows the results of FACS for a cell population obtained by inducing differentiation of the reporter human iPS cell line (OSR1-GFP/SIX2-tdTomato reporter human iPS cells) using the MET antibody and renal progenitor cell markers OSR1 and SIX2.
Fig. 2 shows immune cell staining images of cells isolated with the anti-MET antibody from the cell population obtained by inducing differentiation of the iPS cell line (OSR1-GFP / SIX2-tdTomato reporter human iPS cells) using the antibody against the renal progenitor cell marker SIX2 (photographed). Results are shown compared to MET-negative cells.

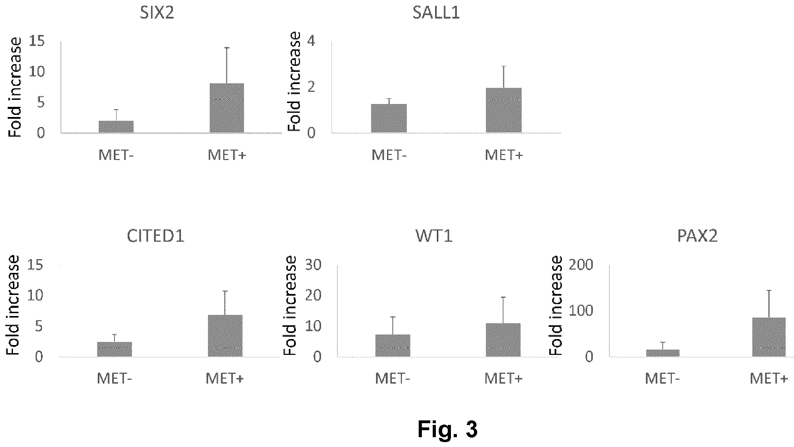

Fig. 3 shows the results of quantitative RT-PCR analysis of expression of nephron progenitor cell marker genes in cells isolated with the anti-MET antibody from the cell population obtained by inducing differentiation of the iPS cell line derived from Alport syndrome patients. Results are shown compared to MET-negative cells.

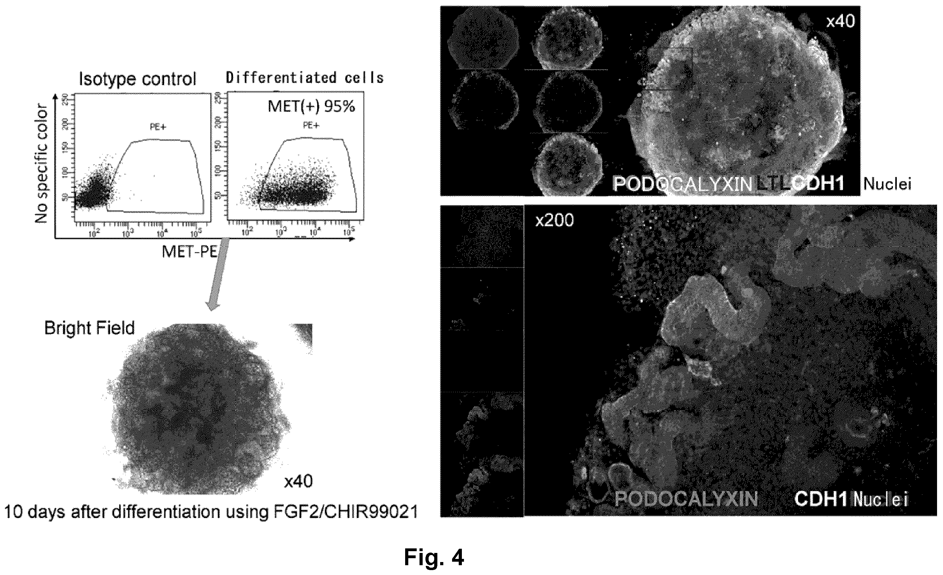

Fig. 4 shows microscopic photographs of renal organoids obtained by culturing renal progenitor cells isolated with the anti-MET antibody (MET (+) 95%) from the cell population obtained by inducing differentiation of the iPS cell line derived from autosomal dominant polycystic kidney disease (ADPKD) patients. The lower left panel is a bright field image of the formed renal organoid. The upper and lower panels on the right are immunostaining images of renal organoids. Green: PODOCALYXIN, glomerular podocyte marker; Red: Lotus tetragonolobus lectin (LTL), proximal tubule marker; White: CDH1, distal tubule marker.

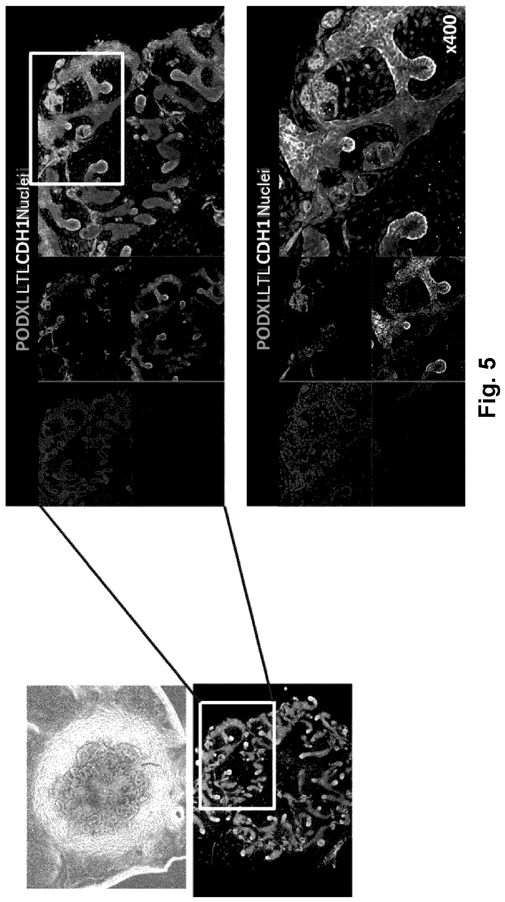

Fig. 5 shows microscopic photographs (Day 13 of differentiation) of renal organoids obtained by culturing renal progenitor cells isolated with the anti-MET antibody from the cell population obtained by inducing differentiation of the iPS cell line derived from Alport syndrome patients. The upper left panel is a bright field image of the formed renal organoid. The lower left panel is an immunostaining image of renal organoids (weak magnification, 100-fold). The upper right panel is a moderately magnified (200-fold) immunostaining images of renal organoids. The lower right panel is a strongly magnified (400-fold) immunostaining images of renal organoids. Green: PODOCALYXIN, glomerular podocyte marker; Red: Lotus tetragonolobus lectin (LTL), proximal tubule marker; White: CDH1, distal tubule marker.

### Description of Embodiments

The present invention will be described in detail below.

### <Method for Concentrating Renal Progenitor Cells>

The method for concentrating renal progenitor cells of the invention comprises the step of extracting MET-positive cells and/or AGTR2-positive cells from a cell population containing renal progenitor cells. The method for concentrating renal progenitor cells of the invention can be paraphrased as a method for sorting renal progenitor cells.

In the present invention, renal progenitor cells can be regarded as cells equivalent to nephron progenitor cells and can differentiate into organ structures such as glomerular structure and tubular structure of the kidney, and their ability to differentiate into organ structures can be evaluated by the method described in, for example, Osafune K, et al. (2006), Development 133: 151-61. SIX2 is known as a characteristic factor for maintaining the state as renal progenitor cells (Cell Stem Cell 3: 169-181 (2008)), and SIX2-positive renal progenitor cells can be mentioned as examples of renal progenitor cells. For example, pluripotent stem cells having a reporter gene (e.g., tdTomato) introduced under the control of the SIX2 promoter (e.g., "OSR1-GFP/SIX2-tdTomato reporter human iPS cells" described in the Examples below) are cultured, and SIX2-positive renal progenitor cells can be isolated by a method known in the art (e.g., a method using a cell sorter) using the expression of the reporter gene as an index. In addition, the expression of SIX2 in renal progenitor cells can be confirmed by a method for analyzing gene expression such as quantitative RT-PCR (Nat Commun 4,1367, (2013)). In the present invention, SIX2-positive renal progenitor cells include cells expressing the SIX2 protein and cells expressing the protein encoded by the gene under the control of the SIX2 promoter. SIX2 includes a gene having the nucleotide sequence described in NM_016932.4 in humans and NM_011380.2 in mice as an accession number of NCBI, a protein encoded by the gene, and a naturally occurring mutant having these functions. Preferably, renal progenitor cells induced by the method of the present invention are OSR1- and SIX2-positive cells, more preferably HOX11-, PAX2-, CITED1-, WT1-, and SALL1-positive cells. Positive expression of these markers, as well as MET and/or AGTR2, can be confirmed by cell (tissue) staining with antibodies against these proteins, cell sorting, or quantitative PCR on these mRNAs.

In the present invention, the origin of a "cell population containing renal progenitor cells" is not particularly limited as long as it is a population of cells containing renal progenitor cells. For example, the cell population may be a cell population contained in isolated renal tissue or a cell population containing renal progenitor cells obtained by differentiation induction from pluripotent stem cells.

In the present invention, concentration of renal progenitor cells means that the proportion of renal progenitor cells is increased as compared with that before the extraction operation, and preferably, renal progenitor cells are concentrated such that the content thereof is 50% or more, 60% or more, 70% or more, 80% or more, or 90% or more. More preferably, cells consisting of 100% renal progenitor cells can be obtained. In other words, the present invention provides a cell population containing renal progenitor cells that are positive for MET and/or AGTR2 expression and positive for OSR1 and/or SIX2 (preferably further positive for one or more of HOX11, PAX2, CITED1, WT1, and SALL1) at 50% or more, 60% or more, 70% or more, 80% or more, or 90% or more.

MET is a receptor for hepatocyte growth factor (HGF) and is also called cMET. In the case of humans, for example, a protein having an amino acid sequence encoded by the base sequence of National Center for Biotechnology Information (NCBI) GenBank Accession No. NM_001127500.2, NM_000245.3, NM_001324402.1, or NM_001324401.1 or its homolog can be mentioned. In the case of mice, for example, a protein having an amino acid sequence encoded by the base sequence of NCBI GenBank Accession No. NM_008591.2 or its homolog can be mentioned.

AGTR2 is a receptor for angiotensin (type 2). In the case of humans, for example, a protein having an amino acid sequence encoded by the base sequence of NCBI GenBank Accession No. NM_000686.4 or its homolog can be mentioned. In the case of mice, for example, a protein having an amino acid sequence encoded by the base sequence of NCBI GenBank Accession No. NM_007429.5 or its homolog can be mentioned.

When extracting renal progenitor cells using MET-positive and/or AGTR2-positive as an index, the expression of MET protein and/or AGTR2 protein may be used as an index, or the expression of the gene (mRNA expression) encoding the MET protein and/or AGTR2 protein may be used as an index. In a case in which isoforms generated by alternative splicing exist for the MET gene and the AGTR2 gene, those isoforms are also included in the category of the MET gene and the AGTR2 gene.

In the present invention, since it was first found that MET and AGTR2 are expressed in renal progenitor cells, renal progenitor cells can be efficiently concentrated using MET positivity and/or AGTR2 positivity as an index. A specific concentration method will be described later.

In the present invention, in a case in which renal progenitor cells are extracted from a cell population containing renal progenitor cells using MET positivity and/or AGTR2 positivity as an index, they can be used in combination with other renal progenitor cell markers as described in Patent Literature 1. In a case in which MET and AGTR2, and even other renal progenitor cell markers are used in an arbitrary combination, the concentration rate of renal progenitor cells is increased as compared with the case of using them alone.

Hereinafter, a method for inducing differentiation of a cell population containing renal progenitor cells from pluripotent stem cells will be described.

<Pluripotent Stem Cells>

The pluripotent stem cells that can be used in the present invention to obtain a cell population containing renal progenitor cells are stem cells that have pluripotency that allows differentiation into all cells present in the living body and proliferative ability, which include, but are not limited to, for example, embryonic stem (ES) cells, male germline stem cells ("GS cells"), embryonic germ cells ("EG cells"), induced pluripotent stem (iPS) cells, and embryonic stems derived from cloned embryos obtained by nuclear transplantation (ntES) cells. Preferred pluripotent stem cells are ES cells and iPS cells.

### (A) Embryonic Stem Cells

ES cells are pluripotent and self-renewing proliferative stem cells established from the inner cell mass of the early embryo (e.g., blastocyst) of a mammal such as a human or a mouse.

ES cells are embryo-derived stem cells from the inner cell mass of the blastocyst, which is the embryo after the morula at the 8-cell stage of a fertilized egg, and have ability to differentiate into all cells that constitute the living body, so-called pluripotent differentiation and ability to proliferate by self-replication. ES cells were discovered in mice in 1981 (M. J. Evans and M.H. Kaufman (1981), Nature 292:154-156), after which ES cell lines were established in primates such as humans and monkeys (J. A. Thomson et al. (1998), Science 282:1145-1147; J. A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165).

Human and monkey ES cells are described, for example, in USP5,843,780; Thomson JA, et al. (1995), Proc Natl. Acad. Sci. U S A. 92:7844-7848; Thomson JA, et al. (1998), Science. 282:1145-1147; H. Suemori et al. (2006), Biochem. Biophys. Res. Commun., 345: 926-932; M. Ueno et al. (2006), Proc. Natl. Acad. Sci. USA, 103:9554-9559; H. Suemori et al. (2001), Dev. Dyn., 222:273-279; H. Kawasaki et al. (2002), Proc. Natl. Acad. Sci. USA, 99:1580-1585; and Klimanskaya I, et al. (2006), Nature. 444:481-485.

Human ES cell lines are available, for example, WA01 (HI) and WA09 (H9) from the WiCell Research Institute, and KhES-1, KhES-2, and KhES-3 from the Institute for Frontier Life and Medical Sciences, Kyoto University (Kyoto, Japan).

### (B) Male Germline Stem Cells

Male germline stem cells are testis-derived pluripotent stem cells, which are the origin cells for spermatogenesis. Similar to ES cells, these cells can be induced to differentiate into cells of various lineages, and have properties of, for example, being able to produce a chimeric mouse when transplanted into a mouse blastocyst (M. Kanatsu-Shinohara et al. (2003) Biol. Reprod., 69:612-616; K. Shinohara et al. (2004), Cell, 119:1001-1012). They can self-renew in a culture medium containing a glial cell line-derived neurotrophic factor (GDNF), and male germline stem cells can be obtained by repeating subculture under the same culture conditions as ES cells (Masanori Takebayashi et al. (2008), Experimental Medicine, Vol. 26, No. 5 (special number), pp. 41-46, YODOSHA CO., LTD. (Tokyo, Japan)).

### (C) Embryonic Germ Cells

Embryonic germ cells are cells that are established from embryonic primordial germ cells and have pluripotency similar to ES cells, and can be established by culturing germ primordialgerm cells in the presence of substances such as LIF, bFGF, and stem cell factors (Y. Matsui et al. (1992), Cell, 70:841-847; J.L. Resnick et al. (1992), Nature, 359:550-551).

### (D) Induced Pluripotent Stem Cells

Induced pluripotent stem (iPS) cells are somatic cell-derived artificial stem cells with properties similar to ES cells, such as pluripotent differentiation and self-renewal proliferative capacity, which can be made by introducing a specific reprogramming factor in the form of DNA or a protein into somatic cells (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al., Nat. Biotechnol. 26:101-106 (2008); WO2007/069666). A reprogramming factor may be composed of a gene that is specifically expressed in ES cells or a gene product or non-coding RNA thereof, a gene that plays an important role in maintaining undifferentiated ES cells or a gene product or non-coding RNA thereof, or a low-molecular-weight compound. Examples of genes included in reprogramming factors include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tell, beta-catenin, Lin28b, Sall1, Sall4, Esrrb, Nr5a2, and Tbx3. These reprogramming factors may be used singly or in combination. Examples of a combination of reprogramming factors include those described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26:2467-2474, Huangfu D, et al. (2008), Nat Biotechnol. 26:1269-1275, Shi Y, et al. (2008), Cell Stem Cell, 3, 568-574, Zhao Y, et al. (2008), Cell Stem Cell, 3:475-479, Marson A, (2008), Cell Stem Cell, 3, 132-135, Feng B, et al. (2009), Nat Cell Biol. 11:197-203, R.L. Judson et al., (2009), Nat. Biotech., 27:459-461, Lyssiotis CA, et al. (2009), Proc Natl Acad Sci U S A. 106:8912-8917, Kim JB, et al. (2009), Nature. 461:649-643, Ichida JK, et al. (2009), Cell Stem Cell. 5:491-503, Heng JC, et al. (2010), Cell Stem Cell. 6:167-74, Han J, et al. (2010), Nature. 463:1096-100, and Mali P, et al. (2010), Stem Cells. 28:713-720.

The reprogramming factors described above also include histone deacetylase (HDAC) inhibitors [e.g., low-molecular-weight inhibitors such as valproic acid (VPA), trichostatin A, sodium butyrate, MC 1293, and M344, nucleic acid expression inhibitors such as siRNA and shRNA against HDAC (e.g., HDAC1 siRNA Smartpool (registered trademark) (Millipore) and HuSH 29mer shRNA Constructs against HDAC1 (OriGene)), and the like], MEK inhibitors (e.g., PD184352, PD98059, U0126, SL327, and PD0325901), glycogen synthase kinase-3 inhibitors (e.g., Bio and CHIR99021), DNA methyltransferase inhibitors (e.g., 5-azacytidine), gistone methyltransferase inhibitors (e.g., low-molecular-weight inhibitors such as BIX-01294, nucleic acid expression inhibitors such as siRNA and shRNA against Suv39hl, Suv39h2, SetDB1, and G9a, and the like), L-channel calcium agonists (e.g., Bayk8644), butyric acid, TGFβ inhibitors or ALK5 inhibitors (e.g., LY364947, SB431542, 616453, andA-83-01), p53 inhibitors (e.g., siRNA and shRNA against p53), ARID3A inhibitors (e.g., siRNA and shRNA against ARID3A), miRNAs such as miR-291-3p, miR-294, miR-295, and mir-302, Wnt Signaling (e.g., soluble Wnt3a), Neuropeptide Y, prostaglandins (e.g., prostaglandin E2 and prostaglandin J2), and factors used for the purpose of increasing establishment efficiency such as hTERT, SV40LT, UTF1, IRX6, GLIS1, PITX2, and DMRTB1. Unless otherwise specified herein, the factors used for the purpose of improving the establishment efficiency of those factors shall not be distinguished from the reprogramming factors.

In a case in which a reprogramming factor is in the protein form, it may be introduced into somatic cells by a technique such as lipofection, fusion with cell membrane-penetrating peptides (e.g., HIV-derived TAT and polyarginine), or microinjection.

Meanwhile, in a case in which a reprogramming factor is in the DNA form, it can be introduced into somatic cells by, for example, a vector such as a virus, a plasmid, or an artificial chromosome, or a technique such as lipofection, liposome, or microinjection. Examples of virus vectors include retroviral vector, lentiviral vector (Cell, 126, pp.663-676, 2006; Cell, 131, pp.861-872, 2007; Science, 318, pp.1917-1920, 2007), adenovirus vector (Science, 322, 945-949, 2008), adeno-associated virus vector, Sendai virus vector (WO2010/008054). Further, examples of an artificial chromosome vector include a human artificial chromosome (HAC), a yeast artificial chromosome (YAC), and bacterial artificial chromosomes (BAC, PAC). As the plasmid, a plasmid for mammalian cells can be used (Science, 322:949-953, 2008). The vector can contain regulatory sequences such as a promoter, an enhancer, a ribosome binding sequence, a terminator, a polyadenylation sites, and the like such that the nuclear reprogramming substance can be expressed. Further, if necessary, it may contain selectable marker sequences of drug resistance genes (e.g., canamycin resistance gene, ampicillin resistance gene, and puromycin resistance gene), thymidine kinase gene, diphtheriatoxin gene, and the like, and reporter gene sequences of green fluorescent protein (GFP), β-glucuronidase (GUS), FLAG, and the like. In addition, the above-described vector may have LoxP sequences before and after the gene encoding the reprogramming factor or the promoter and the gene encoding the reprogramming factor that binds to the gene in order to cleave them after introduction into somatic cells.

In the case of RNA form, it may be introduced into somatic cells by a method such as lipofection or microinjection, and in order to suppress degradation, RNA incorporating 5-methylcytidine and pseudouridine (TriLink BioTechnologies, Inc.) may be used (Warren L, (2010) Cell Stem Cell. 7:618-630).

Examples of culture medium for iPS cell induction include a DMEM, DMEM/F12, or DME culture medium containing 10%-15% FBS (these culture media may also include LIF, penicillin/streptomycin, puromycin, L-glutamine, non-essential amino acids, β-mercaptoethanol, and the like if appropriate), or commercially available culture media [e.g., mouse ES cell culture medium (TX-WES culture medium, Thromb-X), primate ES cell culture medium (primate ES/iPS cell culture medium, ReproCELL Inc.), serum-free medium (mTeSR, STEMCELL Technologies Inc.)] and the like.

As an example of the culture method, for example, in the presence of 5% CO₂ at 37°C, the somatic cells are brought into contact with the reprogramming factor on DMEM or DMEM/F12 culture medium containing 10% FBS and cultured for about 4 to 7 days. Then, the cells are re-sown on feeder cells (e.g., mitomycin C-treated STO cells, SNL cells, or the like), and about 10 days after the contact between the somatic cells and the reprogramming factor, culture is started using the culture medium for bFGF-containing primate ES cell culture. Thus, iPS-like colonies can be generated in about 30 to about 45 days or more after the contact.

Alternatively, in the presence of 5% CO₂ at 37°C, culture is performed on feeder cells (e.g., mitomycin C-treated STO cells, SNL cells, or the like) using DMEM culture medium containing 10% FBS (which may further contain LIF, penicillin/streptomycin, puromycin, L-glutamine, non-essential amino acids, β-mercaptoethanol, and the like), and ES-like colonies can be generated in about 25 to about 30 days or more. Desirably, methods in which instead of feeder cells, the reprogrammed somatic cells themselves are used (Takahashi K, et al. (2009), PLoS One. 4:e8067 or WO2010/137746), or extracellular matrix (e.g., Laminin-5 (WO2009/123349) and Matrigel (BD Falcon)) are exemplified.

In addition to this, a method of culturing using a serum-free medium is also exemplified (Sun N, et al. (2009), Proc Natl Acad Sci USA. 106:15720-15725). Furthermore, in order to increase the establishment efficiency, iPS cells may be established under hypoxic conditions (oxygen concentration of from 0.1% to 15%) (Yoshida Y, et al. (2009), Cell Stem Cell. 5:237-241, or WO2010/013845).

During the above culture, the culture medium is refreshed once daily using fresh culture medium from the second day after the start of culture. The number of somatic cells used for nuclear reprogramming is not limited, but ranges from about 5 × 10³ to about 5 × 10⁶ cells per 100 cm² of cultured dish.

iPS cells can be selected according to the shape of the formed colonies. Meanwhile, in a case in which a drug resistance gene expressed in conjunction with a gene expressed when somatic cells are reprogrammed (e.g., Oct3/4, Nanog) is introduced as a marker gene, established iPS cells can be selected by performing culturing in a culture medium containing the corresponding drug (selected culture medium). In addition, iPS cells can be selected by observing with a fluorescence microscope in a case in which the marker gene is a fluorescent protein gene, by adding a luminescent substrate in a case in which it is a luciferase gene, or by adding a chromogenic substrate in a case in which it is a luciferase gene.

The term "somatic cells" refers to any animal cells (preferably mammalian cell, including human cells) except germline cells such as ova, oocytes, ES cells, or totipotent cells. Somatic cells include, but are not limited to, fetal somatic cells, neonatal somatic cells, and mature healthy or diseased somatic cells, as well as primary cultured cells, subcultured cells, and established cells. Specifically, examples of somatic cells include, for example: (1) tissue stem cells (somatic stem cells) such as nerve stem cells, hematopoietic stem cells, mesenchymal stem cells, and dental pulp stem cells; (2) tissue progenitor cells; (3) differentiated cells such as lymphocytes, epithelial cells, endothelial cells, muscle cells, fibroblasts (e.g., skin cells), hair cells, hepatocytes, gastric mucosal cells, intestinal cells, splenocytes, pancreatic cells (e.g., pancreatic exocrine cells), brain cells, lung cells, renal cells, and fat cells.

An individual mammal from which somatic cells are collected is not particularly limited, but is preferably a human. In a case in which the obtained iPS cells are used for human regenerative medicine, it is particularly preferable that somatic cells are collected from the patient or another person with the same or substantially the same HLA type from the viewpoint of not causing rejection. Here, such "substantially the same" HLA type means that when cells obtained by the differentiation induction from iPS cells derived from the somatic cells are transplanted into a patient by using an immunosuppressant or the like, the HLA types match to an extent that transplanted cells can survive. For example, a case in which the main HLA (e.g., the three loci of HLA-A, HLA-B and HLA-DR) is the same (the same applies hereinafter) is exemplified. Meanwhile, in a case in which iPS cells are not administered (transplanted) to a human, for example, in a method of testing the toxicity of a candidate drug to renal progenitor cells, the origin of somatic cells that are the source of the iPS cells is not particularly limited. In a case in which iPS cells are used as a source of cells for screening to assess a patient's drug susceptibility or side effects, it is desirable that somatic cells from the patient or another person with the same genetic polymorphism that correlates with drug susceptibility or side effects are collected.

Examples of the above-described patient include patients with renal disease, and specifically patients with autosomal dominant polycystic kidney disease (ADPKD) and patients with Alport syndrome.

### (E) ES Cells Derived from Cloned Embryos Obtained by Nuclear Transplantation

nt ES cells are ES cells derived from cloned embryos produced by nuclear transplantation technology and have almost the same characteristics as ES cells derived from fertilized eggs (T. Wakayama et al. (2001), Science, 292: 740-743; S. Wakayama et al. (2005), Biol. Reprod., 72:932-936; J. Byrne et al. (2007), Nature, 450:497-502). In other words, ES cells established from the inner cell mass of a blastocyst derived from a cloned embryo obtained by replacing the nucleus of an unfertilized egg with the nucleus of a somatic cell are nt ES (nuclear transfer ES) cells.

### <Method for Producing Cell Population Containing Renal Progenitor Cells from Pluripotent Stem Cells>

The method for inducing differentiation of pluripotent stem cells into a cell population containing renal progenitor cells is not particularly limited, and known methods can be used. For example, the method disclosed in Patent Literature 1 can be mentioned.

More preferably, a method including the following steps of inducing intermediate mesoderm cells from pluripotent stem cells and further inducing them into renal progenitor cells can be mentioned.

### <Differentiation Induction from Pluripotent Stem Cells to Intermediate Mesoderm Cells>

In inducing the differentiation of pluripotent stem cells into intermediate mesoderm cells, a method including the following steps (i) to (v) can be used.
(i) culturing pluripotent stem cells in a medium containing FGF2, bone morphogenetic protein (BMP) 4, a GSK-3β inhibitor, and retinoic acid or a derivative thereof;
(ii) culturing the cells obtained in the step (i) in a medium containing FGF2, a GSK-3β inhibitor, and BMP7;
(iii) culturing the cells obtained in the step (ii) in a medium containing FGF2, a GSK-3β inhibitor, BMP7, and a TGFβ inhibitor;
(iv) culturing the cells obtained in the step (iii) in a medium containing FGF2, a GSK-3β inhibitor, BMP7, activin, and a ROCK inhibitor; and
(v) culturing the cells obtained in the step (iv) in a medium containing retinoic acid or a derivative thereof and FGF9.

Each step will be further described below.

### (i) Step of culturing pluripotent stem cells in a medium containing FGF2, BMP4, a GSK-3β inhibitor, and retinoic acid or a derivative thereof

In this step, late posterior epiblast is induced from pluripotent stem cells. Late posterior epiblast is characterized as cells that are positive for at least one or more markers of CDX1, OCT4, NANOG, and E-CDH (CDH1), preferably all of these markers are characterized as positive cells. Late posterior epiblast is further preferably negative for EOMES and BRACHYURY.

In step (i), pluripotent stem cells are separated by any method known in the art and preferably adherent-cultured.

Examples of a method for separating pluripotent stem cells include mechanical separation and separation using a separation solution having protease and collagenase activity (e.g., Accutase (trademark) and Accumax (trademark) (Innovative Cell Technologies, Inc)) or a separation solution having only collagenase activity. A preferred method includes causing dissociation using a separation solution having protease activity and collagenase activity, thereby mechanically finely dispersing the cells into single cells. As human pluripotent stem cells used in step (i), it is preferable to use colonies cultured until the dishes used are 70% to 80% confluent.

The medium used in step (i) can be prepared by adding FGF2, BMP4, a GSK-3β inhibitor, and retinoic acid or a derivative thereof to the basal medium used for culturing animal cells. As the basal medium, the basal medium as described above can be used, and may contain serum or may be serum-free. If necessary, serum substitutes, lipids, amino acids, vitamins, growth factors, low-molecular-weight compounds, antibiotics, antioxidants, pyruvic acid, buffers, inorganic salts, and the like may be contained.

As the GSK-3β inhibitor used in step (i), the GSK-3β inhibitor exemplified in the above-described renal progenitor cell differentiation induction step can be used, and CHIR99021 is mentioned as a preferable GSK-3β inhibitor. The concentration of the GSK-3β inhibitor used in step (i) can be appropriately selected by those skilled in the art depending on the GSK-3β inhibitor used, and is, for example, from 0.01 µM to 100 µM, preferably from 0.1 µM to 10 µM, more preferably from 0.5 µM to 3 µM, and particularly preferably from 0.5 µM to 1.5 µM.

FGF2 (basic FGF: bFGF) used in step (i) is preferably human FGF2, and as the human FGF2, for example, a protein having the amino acid sequence of NCBI (National Center for Biotechnology Information) Accession Number: ABO43041.1 is exemplified. FGF2 includes fragments and functional variants thereof as long as it has differentiation-inducing activity. A commercially available FGF2 may be used, and proteins purified from cells or genetically modified proteins may be used. The concentration of FGF2 used in this step is from 1 ng/ml to 1000 ng/ml, preferably from 10 ng/ml to 500 ng/ml, more preferably from 50 ng/ml to 250 ng/ml.

BMP4 used in step (i) is preferably human BMP4, and as the human BMP4, for example, a protein having the amino acid sequence of NCBI (National Center for Biotechnology Information) Accession Number: AAH20546.1 is exemplified. BMP4 includes fragments and functional variants thereof as long as it has differentiation-inducing activity. A commercially available BMP4 may be used, and proteins purified from cells or genetically modified proteins may be used. The concentration of BMP4 used in this step is from 0.1 ng/ml to 100 ng/ml, preferably from 0.5 ng/ml to 50 ng/ml, more preferably from 0.5 ng/ml to 5 ng/ml.

The retinoic acid used in step (i) may be retinoic acid itself or a retinoic acid derivative that retains the differentiation-inducing function of natural retinoic acid. Examples of a retinoic acid derivative include 3-dehydroretinoic acid, 4-[[(5,6,7,8-tetrahydro-5,5,8,8-tetramethyl-2-naphthalenyl)carbonyl]amino]-benzoic acid (AM580) (Tamura K, et al., Cell Differ. Dev. 32:17-26 (1990)), 4-[(1E)-2-(5,6,7,8-tetrahydro-5,5,8,8-tetramethyl-2-naphthalenyl)-1-propen-1-yl]-benzoic acid (TTNPB) (StricklandS, et al., Cancer Res.43:5268-5272 (1983)), compounds described in Tanenaga, K.et al., Cancer Res.40:914-919 (1980), retinol palmitate, retinol, retinal, 3-dehydroretinol, 3-dehydroretinal, and the like.

The concentration of retinoic acid or a derivative thereof used in step (i) is, for example, from 1 nM to 100 nM, preferably from 5 nM to 50 nM, more preferably from 5 nM to 25 nM.

In step (i), the culture temperature is, but is not limited to, from about 30°C to 40°C, preferably about 37°C, and the culture is carried out in an atmosphere of CO₂-containing air. The CO₂ concentration is from about 2% to 5%, preferably about 5%. The culture time in step (i) may be a period sufficient for the differentiation induction to late posterior epiblast, but is, for example, 1 to 2 days, preferably 1 day of culture.

### (ii) Step of culturing the cells obtained in the step (i) in a medium containing FGF2, a GSK-3β inhibitor, and BMP7

In this step, mesoderm lineage primitive streak is induced from late posterior epiblast. The mesoderm lineage primitive streak is characterized as cells positive for CDX1 and BRACHYURY. The mesoderm lineage primitive streak is further preferably negative for OCT4, NANOG, and E-CDH.

In step (ii), the cell population obtained in step (i) may be isolated and adherent-cultured in a separately prepared coated culture dish, or cells obtained by adherent culture in step (i) may be cultured as they are by exchanging the medium.

The medium used in step (ii) can be prepared by adding FGF2, a GSK-3β inhibitor, and BMP7 to the basal medium used for culturing animal cells. As the basal medium, the basal medium as described above can be used, and may contain serum or may be serum-free. If necessary, serum substitutes, lipids, amino acids, vitamins, growth factors, low-molecular-weight compounds, antibiotics, antioxidants, pyruvic acid, buffers, inorganic salts, and the like may be contained.

The FGF2 used in step (ii) is the same as that described in step (i), and its preferred concentration range is also the same.

As the GSK-3β inhibitor used in step (ii), the GSK-3β inhibitor exemplified in step (i) described above can be used, and CHIR99021 is mentioned as a preferable GSK-3β inhibitor. The concentration of the GSK-3β inhibitor used in step (ii) can be appropriately selected by those skilled in the art depending on the GSK-3β inhibitor used, and is, for example, from 0.01 µM to 100 µM, preferably from 0.1 µM to 10 µM, more preferably from 1 µM to 7.5 µM, and particularly preferably from 2 µM to 5 µM. The concentration of the GSK-3β inhibitor used in step (ii) is preferably higher than the concentration in step (i).

BMP7 used in step (ii) is preferably human BMP7, and as the human BMP7, for example, a protein having the amino acid sequence of NCBI (National Center for Biotechnology Information) Accession Number: NM_001719.2 is exemplified. BMP7 includes fragments and functional variants thereof as long as it has differentiation-inducing activity. A commercially available BMP7 may be used, and proteins purified from cells or genetically modified proteins may be used. The concentration of BMP7 used in this step is from 0.1 ng/ml to 100 ng/ml, preferably from 0.5 ng/ml to 50 ng/ml, more preferably from 0.5 ng/ml to 5 ng/ml.

In step (ii), the culture temperature is, but is not limited to, from about 30°C to 40°C, preferably about 37°C, and the culture is carried out in an atmosphere of CO₂-containing air. The CO₂ concentration is from about 2% to 5%, preferably about 5%. The culture time in step (ii) may be a period sufficient for the differentiation induction to mesoderm lineage primitive streak, but is, for example, 10 hours to 2 days or 1 to 2 days, preferably 0.5 to 1 day of culture.

### (iii) Step of culturing the cells obtained in the step (ii) in a medium containing FGF2, a GSK-3β inhibitor, BMP7, and a TGFβ inhibitor;

In this step, late mesoderm lineage primitive streak is induced from the mesoderm lineage primitive streak. The late mesoderm lineage primitive streak is characterized as cells positive for CDX2 and BRACHYURY.

In step (iii), the cell population obtained in step (ii) may be isolated and adherent-cultured in a separately prepared coated culture dish, or cells obtained by adherent culture in step (ii) may be cultured as they are by exchanging the medium.

The medium used in step (iii) can be prepared by adding FGF2, a GSK-3β inhibitor, BMP7, and a TGFβ inhibitor to the basal medium used for culturing animal cells. As the basal medium, the basal medium as described above can be used, and may contain serum or may be serum-free. If necessary, serum substitutes, lipids, amino acids, vitamins, growth factors, low-molecular-weight compounds, antibiotics, antioxidants, pyruvic acid, buffers, inorganic salts, and the like may be contained.

The FGF2, GSK-3β inhibitor, and BMP7 used in step (iii) are the same as in step (ii), and the preferred concentration ranges are also the same, but the concentration range of the GSK-3β inhibitor is from 0.01 µM to 100 µM, preferably from 0.1 µM to 10 µM, more preferably from 1 µM to 7.5 µM, and particularly preferably from 2 µM to 5 µM.

The TGFβ inhibitor used in step (iii) is a substance that inhibits signal transduction from binding of TGFβ to a receptor, leading to SMAD, which is exemplified as a substance that inhibits binding to the ALK family of receptors or a substance that inhibits the phosphorylation of SMAD by the ALK family. Examples thereof include Lefty-1 (e.g., NCBI Accession Nos.: NM_010094 for mice; NM_020997 for humans), SB431542, SB202190 (described in R. K. Lindemann et al., Mol. Cancer, 2003, 2:20), SB505124 (GlaxoSmithKline), NPC30345, SD093, SD908, SD208 (Scios), LY2109761, LY364947, LY580276 (Lilly Research Laboratories), A83-01(WO2009146408), and derivatives thereof. The TGFβ inhibitor can be preferably A83-01.

The concentration of the TGFβ inhibitor in the culture medium is not particularly limited as long as it inhibits ALK, but is from 0.5 µM to 100 µM, preferably from 1 µM to 50 µM, and more preferably from 5 µM to 25 µM.

In step (iii), the culture temperature is, but is not limited to, from about 30°C to 40°C, preferably about 37°C, and the culture is carried out in an atmosphere of CO₂-containing air. The CO₂ concentration is from about 2% to 5%, preferably about 5%. The culture time in step (iii) may be a period sufficient for the differentiation induction to late mesoderm lineage primitive streak, but is, for example, 1 to 3 days, preferably 1.5 to 2 days of culture.

### (iv) Step of culturing the cells obtained in the step (iii) in a medium containing FGF2, a GSK-3β inhibitor, BMP7, activin, and a ROCK inhibitor

In this step, late metanephric lineage primitive streak is induced from the late mesoderm lineage primitive streak. The late metanephric lineage primitive streak is characterized as cells positive for HOX11 and BRACHYURY.

In step (iv), the cell population obtained in step (iii) may be isolated and adherent-cultured in a separately prepared coated culture dish, or cells obtained by adherent culture in step (iii) may be cultured as they are by exchanging the medium.

The medium used in step (iv) can be prepared by adding FGF2, a GSK-3β inhibitor, activin, BMP7, and a ROCK inhibitor to the basal medium used for culturing animal cells. As the basal medium, the basal medium as described above can be used, and may contain serum or may be serum-free. If necessary, serum substitutes, lipids, amino acids, vitamins, growth factors, low-molecular-weight compounds, antibiotics, antioxidants, pyruvic acid, buffers, inorganic salts, and the like may be contained.

The FGF2, GSK-3β inhibitor, and BMP7 used in step (iv) are the same as in step (ii), and the preferred concentration ranges are also the same, but the concentration range of the GSK-3β inhibitor is from 0.01 µM to 100 µM, preferably from 0.1 µM to 10 µM, more preferably form 1 µM to 7.5 µM, and particularly preferably from 2 µM to 5 µM.

The activin used in step (iv) includes activin derived from humans and other animals and functional variants thereof, and for example, commercially available products such as those from R&D Systems, Inc. can be used. The concentration of activin used in step (iv) is from 1 ng/ml to 100 ng/ml, preferably from 5 ng/ml to 50 ng/ml, more preferably from 5 ng/ml to 25 ng/ml.

The ROCK inhibitor used in step (iv) is not particularly limited as long as it can suppress the function of Rho-kinase (ROCK). Examples thereof include Y-27632 (see, for example, Ishizaki et al., Mol. Pharmacol. 57,976-983 (2000); Narumiya et al., Methods Enzymol. 325,273-284 (2000)), Fasudil/HA1077 (see, for example, Uenata et al., Nature 389: 990-994 (1997)), H-1152 (see, for example, Sasaki et al., Pharmacol. Ther. 93: 225-232 (2002)), Wf-536 (see, for example, Nakajima et al., CancerChemother Pharmacol. 52 (4): 319-324 (2003)), and derivatives thereof, as well as antisense nucleic acids against ROCK, RNA interference-inducing nucleic acids (e.g., siRNA), dominant negative mutants, and their expression vectors. In addition, other known low-molecular-weight compounds can also be used as ROCK inhibitors (see, for example: U.S. Patent Publication Nos. 2005/0209261, 2005/0192304, 2004/0014755, 2004/0002508, 2004/0002507, 2003/0125344, and 2003/0087919; and WO2003/062227, WO2003/059913, WO2003/062225, WO2002/076976, and WO2004/039796). In the present invention, one or more ROCK inhibitors can be used. Y-27632 is mentioned as a preferable ROCK inhibitor. The concentration of the ROCK inhibitor used in step (iv) can be appropriately selected by those skilled in the art depending on the ROCK inhibitor used, and is, for example, from 0.1 µM to 100 µM, preferably from 1 µM to 75 µM, and more preferably from 5 µM to 50 µM.

In step (iv), the culture temperature is, but is not limited to, from about 30°C to 40°C, preferably about 37°C, and the culture is carried out in an atmosphere of CO₂-containing air. The CO₂ concentration is from about 2% to 5%, preferably about 5%. The culture time in step (iv) may be a period sufficient for the late metanephric lineage primitive streak to be induced to differentiate, but is, for example, 1 to 5 days, preferably 3 days of culture.

### (v) Step of culturing the cells obtained in the step (iv) in a medium containing retinoic acid or a derivative thereof and FGF9

In this step, late posterior intermediate mesoderm is induced from the late metanephric lineage primitive streak. The intermediate mesoderm is characterized as cells positive for OSR1, HOX11, and WT1.

In step (v), the cell population obtained in step (iv) may be isolated and adherent-cultured in a separately prepared coated culture dish, or cells obtained by adherent culture in step (iv) may be cultured as they are by exchanging the medium.

The medium used in step (v) can be prepared by adding retinoic acid or a derivative thereof and FGF9 to the basal medium used for culturing animal cells. As the basal medium, the basal medium as described above can be used, and may contain serum or may be serum-free. If necessary, serum substitutes, lipids, amino acids, vitamins, growth factors, low-molecular-weight compounds, antibiotics, antioxidants, pyruvic acid, buffers, inorganic salts, and the like may be contained.

The retinoic acid or a derivative thereof used in step (v) is as described in step (i), and the preferable concentration range thereof is also the same.

FGF9 used in step (v) is preferably human FGF9, and as the human FGF9, for example, a protein having the amino acid sequence of NCBI (National Center for Biotechnology Information) Accession Number: NP_002001.1 is exemplified. FGF9 includes fragments and functional variants thereof as long as it has differentiation-inducing activity. Commercially available FGF9 may be used, and proteins purified from cells or genetically modified proteins may be used. The concentration of FGF9 used in this step is, for example, from 1 ng/ml to 1000 ng/ml, preferably from 10 ng/ml to 500 ng/ml, more preferably from 50 ng/ml to 500 ng/ml, particularly preferably from 100 ng/ml to 300 ng/ml.

The medium used in step (v) may further contain a BMP inhibitor.

Examples of the BMP inhibitor include protein inhibitors such as Chordin, NOGGIN, and Follistatin, Dorsomorphin (i.e., 6-[4-(2-piperidin-1-yl-ethoxy)phenyl]-3-pyridin-4-yl-pyrazolo[1,5-a]pyrimidine) or a derivative thereof (P. B. Yu et al. (2007), Circulation, 116:II_60; P.B. Yu et al. (2008), Nat. Chem. Biol., 4:33-41; J. Hao et al. (2008), PLoS ONE, 3(8):e2904), and LDN193189 (i.e., 4-(6-(4-(piperazin-1-yl)phenyl)pyrazolo[1,5-a]pyrimidin-3-yl)quinoline).

The BMP inhibitor is more preferably NOGGIN, the concentration of which can be, for example, from 1 ng/ml to 100 ng/ml.

In step (v), the culture temperature is, but is not limited to, from about 30°C to 40°C, preferably about 37°C, and the culture is carried out in an atmosphere of CO₂-containing air. The CO₂ concentration is from about 2% to 5%, preferably about 5%. The culture time in step (v) may be a period sufficient for the late posterior intermediate mesoderm to be induced to differentiate, but is, for example, 1 to 3 days, preferably 2 days of culture.

### <Differentiation Induction of Intermediate Mesoderm Cells to Renal Progenitor Cells>

Induction of differentiation of intermediate mesoderm cells into renal progenitor cells can be carried out, for example, by a step of culturing intermediate mesoderm cells as obtained in step (v) in a medium containing a GSK-3β inhibitor and FGF9 (also referred to as step (vi) of renal progenitor cell differentiation induction).

The medium used in the renal progenitor cell differentiation induction step can be prepared by adding a GSK-3β inhibitor and FGF9 to a basal medium used for culturing animal cells. Examples of the basal medium include IMDM medium, Medium 199 medium, Eagle's Minimum Essential Medium (EMEM), αMEM medium, Dulbecco's Modified Eagle's Medium (DMEM), Ham'sF12 (F12) medium, RPMI 1640 medium, Fischer's medium, and mixed media thereof. The medium may contain serum (e.g., fetal bovine serum (FBS)) or may be serum-free. If necessary, for example, one or more serum substitutes such as albumin, transferrin, KnockOut Serum Replacement (KSR) (serum substitute for ES cell culture) (Invitrogen), N2 supplement (Invitrogen), B27 supplement (Invitrogen), fatty acid, insulin, collagen precursors, trace elements, 2-mercaptoethanol, and 3'-thiolglycerol may be contained. Alternatively, one or more substances such as lipids, amino acids, L-glutamine, GlutaMAX (Invitrogen), non-essential amino acids (NEAA), vitamins, growth factors, antibiotics, antioxidants, pyruvate, buffers, inorganic salts, and their equivalents may also be contained. A medium pre-optimized for stem cell culture, such as ReproFF2 (ReproCELL Inc.), may be used. In addition, the medium used in the renal progenitor cell differentiation induction step may contain a ROCK inhibitor such as Y-27632 described later.

The GSK-3β inhibitor used in the renal progenitor cell differentiation induction step is not particularly limited as long as it can inhibit the function of GSK-3β, for example, kinase activity. Examples thereof include Indirubin derivative BIO (also known as GSK-3β inhibitor IX; 6-bromoindirubin-3'-oxime), Maleimide derivative SB216763 (3- (2,4-dichlorophenyl)-4- (1-methyl-1H-indole-3-yl)-1H-pyrrole-2,5-dione), GSK-3β inhibitor VII which is a phenyl-α-bromomethyl ketone compound (α,4-dibromoacetophenone), L803-mts which is a cell-penetrating phosphorylated peptide (also known as GSK-3β peptide inhibitor; Myr-N-GKEAPPAPPQSpP-NH₂), and CHIR99021 having high selectivity (Nature (2008)453:519-523). These compounds can be obtained from, for example, Stemgent, Calbiochem, Biomol, and the like, or may be synthesized. CHIR99021 is mentioned as a preferable GSK-3β inhibitor used in this step. The concentration of the GSK-3β inhibitor used in this step can be appropriately selected by those skilled in the art depending on the GSK-3β inhibitor used, and is, for example, from 0.01 µM to 100 µM, preferably from 0.1 µM to 10 µM, more preferably from 0.5 µM to 3 µM, and particularly preferably from 0.5 µM to 1.5 µM.

FGF9 used in the renal progenitor cell differentiation induction step is preferably human FGF9, and as the human FGF9, for example, a protein having the amino acid sequence of NCBI (National Center for Biotechnology Information) Accession Number: NP_002001.1 is exemplified. FGF9 includes fragments and functional variants thereof as long as it has differentiation-inducing activity. A commercially available FGF9 may be used, and proteins purified from cells or genetically modified proteins may be used. The concentration of FGF9 used in this step is, for example, from 1 ng/ml to 1000 ng/ml, preferably from 10 ng/ml to 500 ng/ml, more preferably from 50 ng/ml to 500 ng/ml, particularly preferably from 100 ng/ml to 300 ng/ml.

There is no upper limit to the number of days of culturing in the renal progenitor cell differentiation induction step because long-term culturing does not have a particular influence on the production efficiency of renal progenitor cells, and for example, it can be 2 days or more, 3 days or more, 4 days or more, or 5 days or more. In the renal progenitor cell differentiation induction step, the culture temperature is, but is not limited to, from about 30°C to 40°C, preferably about 37°C. The culture is carried out in an atmosphere of CO₂-containing air, and the CO₂ concentration is preferably from about 2% to 5%

### <Method for Concentrating or Detecting Renal Progenitor Cells>

The reagent used for extracting or detecting renal progenitor cells from a cell population containing renal progenitor cells is not particularly limited as long as it is a reagent that specifically binds to MET or AGTR2, and an antibody, aptamer, peptide, a specifically recognizable compound, or the like, and preferably an antibody or a fragment thereof can be used.

In addition, when investigating the gene expression of these marker genes, primers and probes that hybridize to these marker genes can be used.

The antibody may be a polyclonal or monoclonal antibody. The antibody can be prepared using a technique well known to those skilled in the art (Current protocols in Molecular Biology edit. Ausubel et al. (1987) Publish. John Wiley and Sons. Section 11.12-11.13). Specifically, when the antibody is a polyclonal antibody, an MET or AGTR2 protein expressed and purified in *Escherichia coli* or the like according to a conventional method, or an oligopeptide having a partial amino acid sequence is synthesized to immunize a non-human animal such as a domestic rabbit, and thus, an antibody can be obtained from the serum of the immunized animal according to a conventional method. Meanwhile, in the case of a monoclonal antibody, an antibody can be obtained from hybridoma cells prepared by cell fusion of spleen cells and myeloma cells obtained from the above-mentioned immunized non-human animal (Current protocols in Molecular Biology edit. Ausubel et al.(1987) Publish. John Wiley and Sons. Section 11.4-11.11). Examples of the antibody fragment include a portion of the antibody (e.g., Fab fragment) or a synthetic antibody fragment (e.g., single-chain Fv fragment "ScFv"). Antibody fragments, such as Fab and F (ab)₂ fragments, can also be made by genetically engineered methods. In a case in which the marker is a membrane protein, it is preferably an antibody against the extracellular domain. For example, as an antibody against MET, an antibody commercially available from R&D Systems, Inc. and the like can be exemplified. Further, as an antibody against AGTR2, an antibody commercially available from Funakoshi Co., Ltd. and the like is exemplified.

To detect or extract bound cells, the MET-binding and AGTR2-binding reagents may be bound or bonded to, for example, a detectable substance such as a fluorescent label, radioactive label, chemiluminescent label, enzyme, biotin, or streptavidin or a substance that enables isolation and extraction of protein A, protein G, beads, magnetic beads, or the like.

The MET binding reagent and the AGTR2 binding reagent may be indirectly labeled. Various methods known to those skilled in the art can be used, including, for example, a method using a pre-labeled antibody (secondary antibody) that specifically binds to an antibody against MET or AGTR2.

The method for detecting renal progenitor cells includes, for example, using a flow cytometer or a technique for separately detecting cells after isolation and purification (e.g., protein chip).

Examples of a method for extracting renal progenitor cells include a method for bonding large particles to the MET binding reagent or AGTR2-binding reagent and precipitating them, a method for magnetically selecting cells using magnetic beads (e.g., MACS), a method for using a cell sorter with a fluorescent label, an antibody, or the like, and a method using an immobilized carrier (e.g., a cell enrichment column).

In the invention, renal progenitor cells can be extracted by selectively collecting cells positive for MET and/or AGTR2 in a cell population containing renal progenitor cells. In the invention, selectively collecting (extracting) cells positive for MET and/or AGTR2 may be collecting (extracting) all cells positive for MET and/or AGTR2. Alternatively, it may be collecting (extracting) cells with MET and/or AGTR2 expression levels above a certain level. For example, it is possible to select cells within the top 50%, cells within the top 40%, cells within the top 33%, cells within the top 30%, cells within the top 20%, or cells within the top 10% in a cell population containing renal progenitor cells in terms of the expression level of the marker.

### <Kit>

The invention provides a kit for concentrating (extracting) or detecting renal progenitor cells, which comprises a reagent that specifically binds to MET and/or a reagent that specifically binds to AGTR2. The detection reagent included in this extraction kit is a substance such as an antibody that specifically binds to MET or an antibody that specifically binds to AGTR2 as described above. The extraction kit according to the invention may include a detection reagent for MET and/or AGTR2 and also instructions describing how to use the detection reagent.

### <Use of Renal Progenitor Cells>

The invention also provides renal organoids prepared using renal progenitor cells obtained by the method described above. The production of renal organoids from iPS cells has been reported, for example, in Nature, 526, 564-568 (2015). In the present invention, renal organoids can be obtained by, for example, culturing the renal progenitor cells obtained by the above method to prepare cell clusters, followed by co-culture with feeder cells such as 3T3-Wnt4 cells, mouse fetal spinal cells, or mouse fetal kidney cells, or by performing semi-gas phase culture using a basal medium containing GSK-3β inhibitor such as CHIR99021 (see Nature, 526, 564-568 (2015)). The medium can contain FGF9 and FGF2 in addition to the GSK-3β inhibitor.

The present invention provides: a pharmaceutical composition containing renal progenitor cells obtained by the method described above or renal organoids obtained using the same.; a therapeutic or prophylactic agent for renal diseases containing the renal progenitor cells or renal organoids obtained by using the renal progenitor cells; and a method for treating or preventing renal disease, which comprises a step of administering a therapeutic or prophylactically effective amount of the renal progenitor cells or renal organoids obtained therefrom. Examples of a method for administering a therapeutic agent to a patient in need of treatment or prophylaxis include: a method for sheeting the obtained renal progenitor cells and attaching them to the patient's kidney; a method for directly transplanting a cell suspension in which the obtained renal progenitor cells are suspended in physiological saline or the like or cell mass obtained by three-dimensionally culturing (e.g., Dev Cell. Sep 11,2012; 23 (3): 637-651) into the patient's kidney; and a method for performing three-dimensional culture on a scaffold composed of Matrigel or the like and transplanting the obtained renal progenitor cell mass. The transplantation site is not particularly limited as long as it is within the kidney, but is preferably under the renal capsule. Examples of renal diseases include acute renal disorder, chronic renal failure, and chronic kidney disease that does not lead to chronic renal failure.

In the present invention, the number of renal progenitor cells contained in the therapeutic agent for renal disease is not particularly limited as long as the graft can survive after administration, and is appropriately increased or decreased according to the size of the affected area and the size of the body.

### Examples

Hereinafter, the present invention will be described in more detail with reference to Examples, but the present invention is not limited to the following Examples.

### <1> Induction of Differentiation of iPS Cells into Renal Progenitor Cells - Acquisition of Cell Population Containing Renal Progenitor Cells

iPS cells were induced to differentiate into Renal progenitor cells using the following protocol. As iPS cells, a 4A6 cell line (OSR1-GFP / SIX2-tdTomato double reporter human iPS cell line) (Stem Cells Transl Med. 2015 Sep; 4(9): 980-992) was used.

1. Unicellularize undifferentiated iPS cells by Accutase treatment, suspend them in ReproFF2 medium (ReproCELL Inc.) supplemented with 10 µM Y27632, and sow the cells at a density of from 1.0 × 10⁴/well to 5.0 × 10⁴/well in wells coated with Matrigel (BD Biosciences), followed incubation for 24 hours at 37°C.
2. Twenty-four hours later (Day 1), replace the medium with a medium supplemented with 1 µM CHIR99021, 10 nM retinoic acid, 1 ng/ml BMP4, and 100 ng/ml FGF2 prepared using DMEM/F12 Glutamax (Thermo Fisher Scientific Inc.) supplemented with vitamin A free B27 supplement (Thermo Fisher Scientific Inc.) as a basal medium. (Preparation of late posterior epiblast (i))
3. On Day 2, replace the medium with a medium prepared by adding 3µM CHIR99021, 1 ng/ml BMP7, and 100 ng/ml FGF2 to the same basal medium. (Preparation of mesoderm lineage primitive streak (ii))
4. On Days 2.5 and 3, replace the medium with a medium prepared by adding 3µM CHIR99021, 1 ng/ml BMP7, 100 ng/ml FGF2, and 10 µM A83-01 to the same basal medium. (Preparation of late mesoderm lineage primitive streak (iii))
5. On Days 4, 5, and 6, replace the medium with a medium prepared by adding 3µM CHIR99021, 1 ng/ml BMP7, 100 ng/ml FGF2, 10 ng/ml ACTIVIN, and 30 µM Y27632 to the same basal medium. (Preparation of late metanephric lineage primitive streak (iv))
6. On Days 7 and 8, replace the medium with a medium prepared by adding 200 ng/ml FGF9, 100 nM retinoic acid, and 25 ng/ml NOGGIN to the same basal medium. (Preparation of late posterior intermediate mesoderm (v))
7. On Days 9, 10, and 11, replace the medium with a medium prepared by adding 200 ng/ml FGF9 and 1 µM CHIR99021 to the same basal medium. (Preparation of renal progenitor cells (vi))

### <2> Concentration of Renal Progenitor Cells Using Anti-MET Antibody from Cell Population Containing Renal Progenitor Cells

1. For renal progenitor cells derived from human iPS cells (prepared by the protocol described in <1>) induced to differentiate by two-dimensional culture on a 24-well plate, after removing the supernatant, perform one wash with 500 µl of PBS.
2. Add 100 µl ofAccumax (Innovative Cell Technologies, Inc., Cat # AM105) and incubate at 37°C for 10 minutes.
3. After neutralization with 900 µl of 10% FBS, count the number of cells.
4. Dispense 1.0 × 10⁶ cells into a 1.5 ml tube and centrifuge at 200 g for 5 minutes.
5. Remove the supernatant and suspend 1.0 × 10⁶ cells in 100 µl of PBS.
6. To the suspension, add 10 µl of fluorescently (APC) labeled MET (HGFR) antibody (R&D systems, Inc. Cat #; FAB3582A) and let stand on ice for 30 minutes (followed by tapping once after 15 minutes).
7. Add 1 ml of PBS (Wash) and centrifuge at 500 g for 5 minutes.
8. Remove the supernatant, add 1 ml of PBS again (Wash), and centrifuge at 500 g for 5 minutes.
9. Remove the supernatant and suspend DAPI in 1 ml of 2% FBS (free of phenol red) adjusted to a final concentration of 1 µg/ml.
10. Perform analysis by flow cytometry (Aria II).

### <3> Preparation of Renal Organoids by Single Culture of Renal Progenitor Cells

An iPS cell line derived from the Alport syndrome patient (CiRA00878-3) or an iPS cell line derived from the ADPKD patient (CiRA00007) was induced to differentiate by the procedures described in <1> above, and a cell population containing renal progenitor cells was obtained. The renal progenitor cells were concentrated using the anti-MET antibody according to the procedures described in <2> above. The concentrated renal progenitor cells were made into a cell mass having a size of 1.0 × 10⁵, and cultured in a basal medium supplemented with 1 µM CHIR99021 and 200 ng/ml FGF9 for 1 to 2 days. The cell mass was subjected to semi-gas phase culture for 2.5 days in a basal medium supplemented with 5 µM CHIR99021 and 200 ng/ml FGF2, and further, semi-gas phase culture was performed in the basal medium alone for 8.5 days.

### <Results>

As shown in Fig. 1, it was found that isolation with the MET antibody efficiently concentrated both OSR1- and SIX2-positive cells, which are renal progenitor cell markers.

In the cell population after induction of differentiation, 19.9% were MET-positive, but by isolating MET-positive cells, 96.9% of them were both OSR1- and SIX2 positive renal progenitor cells. As a result, renal progenitor cells could be concentrated with high efficiency by using MET as a marker.

Fig. 2 shows immunohistochemical staining images with an antibody against the renal progenitor cell marker SIX2 of cells isolated using the MET antibody from the cell population containing the renal progenitor cells obtained in <1>. As a result, it was found that almost all MET-positive cells were SIX2-positive, while almost all MET-negative cells were SIX2-negative. In view of the above, it can be seen that MET is an excellent renal progenitor cell marker.

The expression of the nephron progenitor cell marker gene in renal progenitor cells isolated with the MET antibody from the cell population obtained by inducing differentiation of the iPS cell line derived from the Alport syndrome patient was also analyzed by quantitative RT-PCR. As a result, as shown in Fig. 3, the expression level of MET-positive cells was higher than that of MET-negative cells not only in the nephron progenitor cell marker gene SIX2 but also in SALL1, CITED 1, WT1 and PAX2, thereby confirming that the MET antibody enables efficient concentration of nephron progenitor cells.

In addition, renal progenitor cells isolated with the MET antibody from the cell population obtained by inducing differentiation of the iPS cell line derived from the ADPKD patient or the iPS cell line derived from the Alport syndrome patient were subjected to renal organoid culture. As shown in Figs. 4 and 5, glomeruli and renal tubules could be observed, and therefore, renal organoids were successfully produced.

### <Identification of Marker AGTR2>

The expression levels of genes in renal progenitor cells (OSR1⁺SIX2⁺ cells) and non-renal progenitor cells (OSR1⁻SIX2⁻ cells) were compared to search for genes that were at least twice highly expressed in renal progenitor cells (FC≥2) by GO analysis. As a result, AGTR2 was identified (Table 1). AGTR2 was considered to be available as a marker for renal progenitor cells in the same manner as MET.

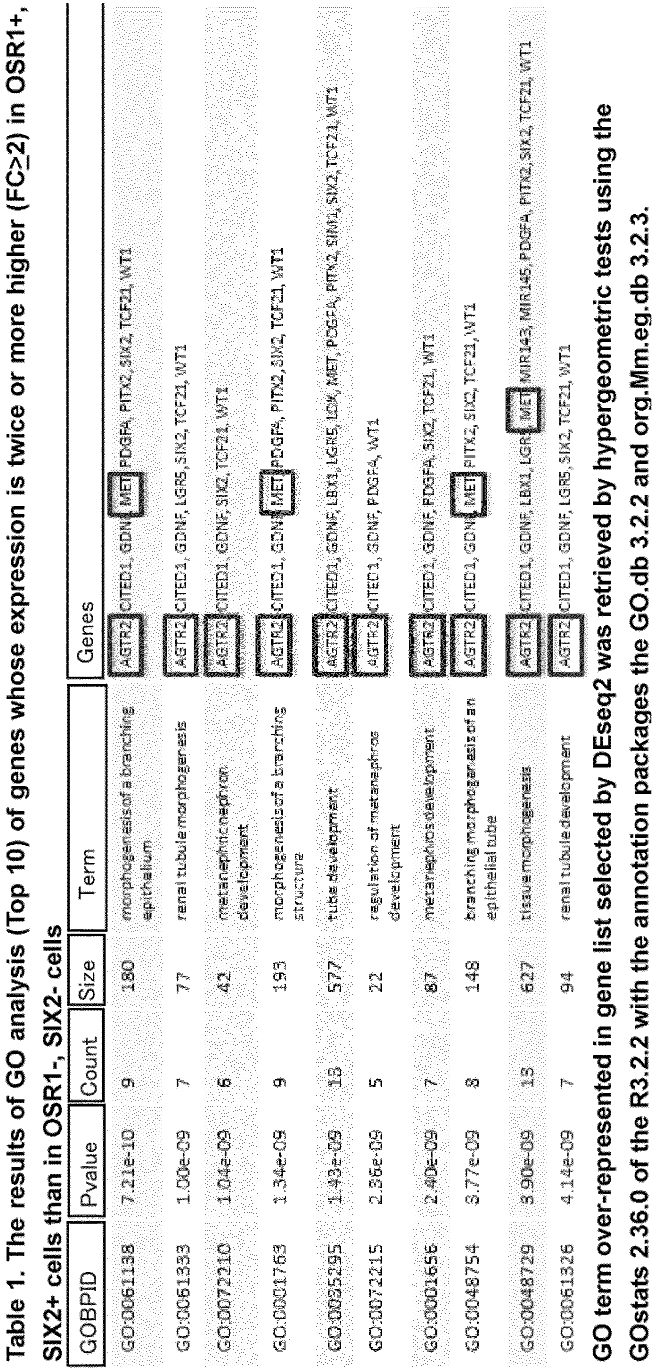

The contents of all documents, including the patents and patent application specifications cited herein, are incorporated herein by reference in their entirety to the same extent as explicitly disclosed. This description includes the disclosure of Japanese Patent Application No. 2018-138040, which is the basis of the priority of the present application.

## Claims

1. A method for concentrating renal progenitor cells, comprising a step of extracting MET-positive cells and/or AGTR2-positive cells from a cell population containing renal progenitor cells.

2. The method according to claim 1, wherein the renal progenitor cells are OSR1- and SIX2-positive.

3. The method according to claim 1 or 2, wherein the step of extracting MET-positive cells and/or AGTR2-positive cells is performed using an anti-MET antibody and/or an anti-AGTR2 antibody.

4. The method according to any one of claims 1 to 3, wherein the renal progenitor cells are human renal progenitor cells.

5. The method according to any one of claims 1 to 4, wherein the cell population containing renal progenitor cells is a cell population containing renal progenitor cells obtained by differentiation induction from pluripotent stem cells.

6. The method according to claim 5, wherein the pluripotent stem cells are ES cells or iPS cells.

7. A method for detecting renal progenitor cells, comprising a step of detecting MET-positive cells and/or AGTR2-positive cells in a cell population containing renal progenitor cells.

8. The method according to claim 7, wherein the renal progenitor cells are OSR1- and SIX2-positive.

9. A kit for extracting or detecting renal progenitor cells, comprising a reagent that specifically binds to MET and/or a reagent that specifically binds to AGTR2.

10. The kit according to claim 9, wherein the renal progenitor cells are OSR1- and SIX2-positive.

11. The kit according to claim 9 or 10, wherein the reagent that specifically binds to MET is an anti-MET antibody, and the reagent that specifically binds to AGTR2 is an anti-AGTR2 antibody.

12. A method for producing renal progenitor cells, comprising: a step of obtaining a cell population containing renal progenitor cells from pluripotent stem cells; and a step of extracting renal progenitor cells from the obtained cell population using MET and/or AGTR2 positivity as an index.

13. The method according to claim 12, wherein the renal progenitor cells are OSR1- and SIX2-positive.

14. The method according to claim 12 or 13, wherein the step of obtaining a cell population containing renal progenitor cells from pluripotent stem cells comprises the following steps (i) to (vi):
(i) culturing pluripotent stem cells in a medium containing fibroblast growth factor (FGF) 2, bone morphogenetic protein (BMP) 4, a glycogen synthase kinase (GSK)-3β inhibitor, and retinoic acid or a derivative thereof;
(ii) culturing the cells obtained in the step (i) in a medium containing FGF2, a GSK-3β inhibitor, and BMP7;
(iii) culturing the cells obtained in the step (ii) in a medium containing FGF2, a GSK-3β inhibitor, BMP7, and a transforming growth factor (TGF) β inhibitor;
(iv) culturing the cells obtained in the step (iii) in a medium containing FGF2, a GSK-3β inhibitor, BMP7, activin, and a Rho-kinase (ROCK) inhibitor;
(v) culturing the cells obtained in the step (iv) in a medium containing retinoic acid or a derivative thereof and FGF9.; and
(vi) culturing the cells obtained in the step (v) in a medium containing a GSK-3β inhibitor and FGF9.

15. A method for producing a renal organoid, comprising: a step of inducing renal progenitor cells by the method according to any one of claims 12 to 14; and a step of culturing the obtained renal progenitor cells to form a renal organoid.

16. A renal progenitor cell marker consisting of MET and/or AGTR2.

17. The renal progenitor cell marker according to claim 16, wherein renal progenitor cells are OSR1- and SIX2-positive.
